# EUROPEAN PATENT SPECIFICATION

(11) **EP 2 703 081 B1**
(45) Date of publication and mention of the grant of the patent: **07.08.2019**
(21) Application number: 12182927.9
(22) Date of filing: 04.09.2012
(51) Int. Cl.: B01J 31/22, B01J 31/18, C07C 6/04

(54) **MOLYBDENUM AND TUNGSTEN COMPLEXES AS OLEFIN METATHESIS CATALYSTS AND REACTIONS USING THE CATALYSTS**
MOLYBDÄN- UND WOLFRAMKOMPLEXE ALS OLEFINMETATHESE-KATALYSATOREN UND REAKTIONEN MIT DEN KATALYSATOREN
COMPLEXES DE MOLYBDÈNE ET DE TUNGSTÈNE COMME CATALYSEURS DE MÉTATHÈSE ET RÉACTIONS UTILISANT CES CATALYSEURS

(43) Date of publication of application: 05.03.2014
(73) Proprietor: XiMo AG, 6048 Horw/Lucerne (CH)
(72) Inventor: Fráter, Georg, Emil, 9642 Ebnat-Kappel (CH); Levente, Ondi, 2112 Veresegyhaz (HU)
(74) Representative: Tostmann, Holger Carl

(56) References cited:
- US-A1- 2011 015 430
- US-A1- 2011 077 421
- RICHARD R. SCHROCK: "Recent Advances in High Oxidation State Mo and W Imido Alkylidene Chemistry", CHEMICAL REVIEWS, vol. 109, no. 8, 13 March 2009 (2009-03-13), pages 3211-3226, XP055063847, ISSN: 0009-2665, DOI: 10.1021/cr800502p
- SMARANDA C MARINESCU ET AL: "Ethenolysis Reactions Catalyzed by Imido Alkylidene Monoaryloxide Monopyrrolide (MAP) Complexes of Molybdenum", JOURNAL OF THE AMERICAN CHEMICAL SOCIETY, ACS PUBLICATIONS, US, vol. 131, no. 31, 12 August 2009 (2009-08-12), pages 10840-10841, XP002643015, ISSN: 0002-7863, DOI: 10.1021/JA904786Y [retrieved on 2009-07-20]
- MIAO YU ET AL: "Enol Ethers as Substrates for Efficient Z - and Enantioselective Ring-Opening/Cross-Metathesis Reactions Promoted by Stereogenic-at-Mo Complexes: Utility in Chemical Synthesis and Mechanistic Attributes", JOURNAL OF THE AMERICAN CHEMICAL SOCIETY, vol. 134, no. 5, 24 January 2012 (2012-01-24), pages 2788-2799, XP055064373, ISSN: 0002-7863, DOI: 10.1021/ja210946z
- BRAD C. BAILEY ET AL: "Evaluation of Molybdenum and Tungsten Metathesis Catalysts for Homogeneous Tandem Alkane Metathesis", ORGANOMETALLICS, vol. 28, no. 1, 12 December 2008 (2008-12-12), pages 355-360, XP055063794, ISSN: 0276-7333, DOI: 10.1021/om800877q
- SMARANDA C. MARINESCU ET AL: "Simple Molybdenum(IV) Olefin Complexes of the Type Mo(NR)(X)(Y)(olefin)", ORGANOMETALLICS, vol. 29, no. 24, 2 December 2010 (2010-12-02), pages 6816-6828, XP055064001, ISSN: 0276-7333, DOI: 10.1021/om101003v
- MIAO YU ET AL: "Synthesis of macrocyclic natural products by catalyst-controlled stereoselective ring-closing metathesis", NATURE, vol. 479, no. 7371, 2 November 2011 (2011-11-02), pages 88-93, XP055064353, ISSN: 0028-0836, DOI: 10.1038/nature10563
- HYANGSOO JEONG ET AL: "Syntheses of Tungsten tert -Butylimido and Adamantylimido Alkylidene Complexes Employing Pyridinium Chloride As the Acid", ORGANOMETALLICS, vol. 31, no. 18, 30 August 2012 (2012-08-30), pages 6522-6525, XP055263092, US ISSN: 0276-7333, DOI: 10.1021/om300799q

## Description

The invention relates to alkene metathesis catalysts. Alkene metathesis (olefin metathesis) is a reaction between alkenes or olefinic groups, in which formally alkylidene groups are exchanged between the alkenes or olefinic groups. Examples of metathesis reactions include cross metathesis, i.e. the reaction between two different olefins forming a new olefin or new olefins, the ring opening metathesis of a cyclic diene, the ring closing metathesis of a diene, the ethenolysis of an olefin having an internal olefinic double bond to form an olefin having a terminal olefinic double bond, and the formation of internal olefin(s) from terminal olefin(s) via homo-metathesis reactions. The latter reaction may be regarded as a cross metathesis between two identical olefins.

US 2011/077421 A1 relates generally to catalysts and processes for the Z-selective formation of internal olefin(s) from terminal olefin(s) via homo-metathesis reactions. The method comprises reacting a first molecule comprising a terminal double bond and a second, identical molecule via a homo-metathesis reaction to produce a product comprising an internal double bond, wherein the internal double bond of the product comprises one carbon atom from the terminal double bond of the first molecule and one carbon atom from the terminal double bond of the second carbon atom, and wherein at least about 60 % of the internal double bond of the product is formed as the Z-isomer. This reaction is catalyzed by a compound of formula 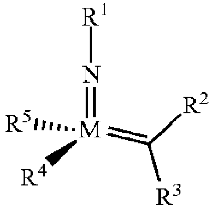 wherein **M** is Mo or W, **R¹** is aryl, heteroaryl, alkyl, heteroalkyl, optionally substituted, **R²** and **R³** can be the same or different and are hydrogen, alkyl, alkenyl, heteroalkyl, heteroalkenyl, aryl, or heteroaryl, optionally substituted, and **R⁴** and **R⁵** can be the same or different and are alkyl, heteroalkyl, aryl, heteroaryl, silylalkyl, or silyloxy, optionally substituted, wherein at least one of **R⁴** or **R⁵** is a ligand containing oxygen bound to **M.** Preferably, bidentate structures may be used as ligand **R⁴.**

Further compounds based on Mo and W useful as catalysts in metathesis reaction are known from US 6,121,473, US 2008/0119678 and US 2011/0015430.

Such catalysts usually have to be applied in a metathesis reaction in a relatively high amount in order to achieve a sufficient degree of conversion of the olefin(s) used as starting material. It is known that a weight ratio up to 1 : 500 with respect to the applied olefin(s) (weight catalyst: weight olefin(s)) is necessary in order to achieve a conversion of 30 % or more. Accordingly, and since these catalysts are relatively costive, such reactions are costive at an industrial scale.

It is an object of the invention to provide compounds useful as metathesis catalysts that perform metathesis in an amount as low as possible with respect to the applied olefin(s) and still allow for a high conversion while optionally providing for a specific stereoselectivity, and further optionally not only allowing for increased formation of Z-isomers in a homo-metathesis reaction, but which may beneficially catalyze other metathesis reactions such as cross metathesis or ethenolysis of an olefin having an internal olefinic bond. Accordingly, the compounds should be able to allow for a conversion in a metathesis reaction of at least 30 % when applied in a weight ratio of less than 1 : 500 with respect to the olefin(s) to be reacted. Such conversion and weight ratio are considered to allow for a beneficial reaction at an industrial scale.

A compound that is suitable as catalyst for said metathesis reaction is of the following formula: 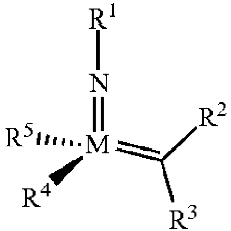 wherein the substituents are defined as in claims 1 or 2.

In one embodiment, **M** is Mo or W; **R¹** is selected from 2,6-dimethylphenyl, 2,6-diisopropylphenyl, 2,6-dichlorophenyl; **R²** is -C(CH₃)₂C₆H₅ or -C(CH₃)₃; **R³** is H; **R⁵** is selected from pyrrol-1-yl and 2,5-dimethyl-pyrrol-1-yl; **R⁴** is **R⁶**-X-, wherein **R⁶**-X- is selected from 4-methyl-2,6-di-t-butylphenoxy or 4-methoxy-2,6-di-t-butylphenoxy or 4-bromo-2,6-di-t-butylphenoxy or 2,4,6-tri-t-butylphenoxy.

In one embodiment, **M** is W; **R¹** is 2,6-dichlorophenyl; **R²** is -C(CH₃)₂C₆H₅ or -C(CH₃)₃; **R³** is H; **R⁵** is pyrrol-1-yl; **R⁴** is 4-bromo-2,6-di-t-butylphenoxy or 2,4,6-tri-t-butylphenoxy or 4-methoxy-2,6-di-t-butylphenoxy.

In one embodiment, **M** is Mo; **R¹** is 2,6-diisopropylphenyl; **R²** is -C(CH₃)₂C₆H₅ or-C(CH₃)₃; **R³** is H; **R⁵** is pyrrol-1-yl; **R⁴** is 4-methyl-2,6-di-t-butylphenoxy or 4-bromo-2,6-dit-butylphenoxy or 2,4,6-tri-t-butylphenoxy or 4-methoxy-2,6-di-t-butylphenoxy.

In one embodiment, **M** is Mo or W; **R¹** is selected from 2,6-dimethylphenyl, 2,6-diisopropylphenyl, 2,6-dichlorophenyl; **R²** is -C(CH₃)₂C₆H₅ or -C(CH₃)₃; **R³** is H; **R⁵** is selected from pyrrol-1-yl and 2,5-dimethyl-pyrrol-1-yl; **R⁴** is **R⁶**-X-,
wherein **R⁶**-X- is selected from 4-chloro-2,6-diphenylphenoxy, 4-bromo-2,6-diphenylphenoxy, 4-fluoro-2,6-diphenylphenoxy, 4-fluoro-2,6-di(2,4,6-trimethylphenyl)phenoxy, or 4-chloro-2,3,5,6-tetraphenylphenoxy or 4-bromo-2,3,5,6-tetraphenylphenoxy or 4-fluoro-2,3,5,6-tetraphenylphenoxy or 4-bromo-3,5-diphenyl-2,6-di(4-bromophenyl)phenoxy.

In one embodiment, **M** is Mo; **R¹** is selected from 2,6-diisopropylphenyl; **R²** is-C(CH₃)₂C₆H₅ or -C(CH₃)₃; **R³** is H; **R⁵** is pyrrol-1-yl; **R⁴** is 4-bromo-2,6-diphenylphenoxy, 4-fluoro-2,6-diphenylphenoxy, or 4-fluoro-2,6-di(2,4,6-trimethylphenyl)phenoxy.

In one embodiment, **M** is Mo; **R¹** is selected from 2,6-dimethylphenyl; **R²** is -C(CH₃)₂C₆H₅ or -C(CH₃)₃; **R³** is H; **R⁵** is 2,5-dimethyl-pyrrol-1-yl; **R⁴** is selected from 4-fluoro-2,6-di(2,4,6-trimethylphenyl)phenoxy.

In one embodiment, **M** is Mo; **R¹** is 2,6-dimethylphenyl; **R²** is -C(CH₃)₂C₆H₅ or -C(CH₃)₃; **R³** is H; **R⁵** is 2,5-dimethyl-pyrrol-1-yl; **R⁴** is selected from 4-bromo-2,6-diphenylphenoxy, and

In one embodiment, **R¹** is 2,6-dimethylphenyl or 2,6-diisopropylphenyl; **R²** is-C(CH₃)₂C₆H₅ or -C(CH₃)₃; **R³** is H; **R⁴** is 4-bromo-2,6-diphenylphenoxy or 4-bromo-2,3,5,6-tetraphenylphenoxy; **R⁵** is pyrrol-1-yl or 2,5-dimethyl-pyrrol-1-yl.

In one embodiment, **M** is Mo; **R¹** is 2,6-dimethylphenyl or 2,6-diisopropylphenyl; **R²** is-C(CH₃)₂C₆H₅ or -C(CH₃)₃; **R³** is H; **R⁴** is 4-bromo-2,6-diphenylphenoxy; **R⁵** is 2,5-dimethyl-pyrrol-1-yl.

In still another embodiment, **M** is Mo; **R¹** is 2,6-dimethylphenyl or 2,6-diisopropylphenyl; **R²** is -C(CH₃)₂C₆H₅ or -C(CH₃)₃; **R³** is H; **R⁴** is 4-bromo-2,3,5,6-tetraphenylphenoxy; **R⁵** is 2,5-dimethyl-pyrrol-1-yl.

In one embodiment, **M** is Mo; **R¹** is 2,6-diisopropylphenyl; **R²** is -C(CH₃)₂C₆H₅ or-C(CH₃)₃; **R³** is H; **R⁴** is 4-methyl-2,6-di-t-butylphenoxy or 4-methoxy-2,6-di-t-butylphenoxy or 4-bromo-2,6-di-t-butylphenoxy or 2,4,6-tri-t-butylphenoxy; **R⁵** is pyrrol-1-yl.

In one embodiment, **M** is W; **R¹** is 2,6-dichlorophenyl; **R²** is -C(CH₃)₃; **R³** is H; **R⁵** is pyrrol-1-yl; **R⁴** is 4-methoxy-2,6-di-t-butylphenoxy or 4-bromo-2,6-di-t-butylphenoxy or 2,4,6-tri-t-butylphenoxy.

Each of these three W-based compounds of the afore-mentioned embodiment, when used as a catalyst in a cross metathesis reaction, may provide for excellent Z-selectivity, which is around 90 % Z and around 10% E.

In one embodiment, **M** is Mo; **R¹** is 2,6-diisopropylphenyl; **R²** is -C(CH₃)₂C₆H₅ or-C(CH₃)₃; **R³** is H; **R⁴** is 4-bromo-2,6-diphenylphenoxy or 4-bromo-2,3,5,6-tetraphenylphenoxy; **R⁵** is pyrrol-1-yl.

In one embodiment, **M** is Mo or W; **R¹** is selected from 2,6-dimethylphenyl, 2,6-diisopropylphenyl, 2,6-dichlorophenyl; **R²** is -C(CH₃)₃; **R³** is H; **R⁵** is selected from pyrrol-1-yl; 2,5-dimethyl-pyrrol-1-yl; **R⁴** is selected from 4-bromo-2,6-diphenylphenoxy, 4-fluoro-2,6-diphenylphenoxy, 4-methyl-2,6-diphenylphenoxy, 2,4,6-triphenylphenoxy, 4-fluoro-dimesitylphenoxy, 4-bromo-2,6-di-tert.-butylphenoxy, 4-methoxy-2,6-di-tert.-butylphenoxy, 4-methyl-2,6-di-tert.-butylphenoxy, 2,4,6-tri-tert.-butylphenoxy, 4-bromo-2,3,5,6-tetraphenylphenoxy, 4-bromo-2,6-di(4-bromophenyl)-3,5-diphenylphenoxy.

The catalysts may be prepared according to known methods or methods known from US 2011/0077421 A1, US 6,121,473, US 2008/0119678 and US 2011/0015430.

The compounds may be advantageously used in metathesis reactions as specified above. Without being bound by theory, it is believed that in particular **R⁴** as defined provides for a high yield and stereoselectivity in the various types of metathesis reactions. Furthermore, the starting materials of the catalysts, in particular the starting materials used to introduce residue **R⁴** into the Mo or W-compound are mostly commercially available or may be simply prepared according to known methods. This makes the novel catalysts particularly applicable for industrial purposes, i.e. for alkene metathesis reactions performed at industrial scale.

Accordingly, said catalysts may advantageously be applied in various types of metathesis reactions.

In one embodiment of the method according to the invention, the first olefin has a terminal olefinic double bond, and the second olefin has a terminal olefinic double bond, wherein the first and the second olefin are identical.

In another embodiment, the first and the second olefin are different from one another.

In still another embodiment, the first olefin has an internal olefinic double bond and the second olefin is ethylene.

In one embodiment, when **M** is Mo; **R¹** is 2,6-diisopropylphenyl; **R²** is -C(CH₃)₂C₆H₅; **R³** is H; **R⁴** is 1-(4-bromo-2,6-diphenylphenoxy); **R⁵** is 2,5-dimethyl-pyrrol-1-yl; or when **M** is Mo; **R¹** is 2,6-dimethylphenyl; **R²** is -C(CH₃)₂C₆H₅; **R³** is H; **R⁴** is 4-bromo-2,3,5,6-tetraphenylphenoxy; **R⁵** is 2,5-dimethyl-pyrrol-1-yl; the catalysts allow for ethenolysis resulting in a high yield of an olefin having a terminal olefinic double bond.

Other preferred compounds have the following structures:
**M** is Mo; **R¹** is 2,6-dimethylphenyl; **R²** is -C(CH₃)₂C₆H₅; **R³** is H; **R⁴** is 4-bromo-2,3-diphenylphenoxy; **R⁵** is 2,5-dimethyl-pyrrol-1-yl; or **M** is Mo; **R¹** is 2,6-diisopropylphenyl; **R²** is -C(CH₃)₂C₆H₅; **R³** is H; **R⁴** is 4-bromo-2,3,5,6-tetraphenylphenoxy; **R⁵** is 2,5-dimethyl-pyrrol-1-yl; or **M** is Mo; **R¹** is 2,6-diisopropylphenyl; **R²** is -C(CH₃)₂C₆H₅; **R³** is H; **R⁴** is 4-methyl-2,3-di-t-butylphenoxy; **R⁵** is pyrrol-1-yl; or **M** is Mo; **R¹** is 2,6-diisopropylphenyl; **R²** is -C(CH₃)₂C₆H₅; **R³** is H; **R⁴** is 4-bromo-2,3-di-t-butylphenoxy; **R⁵** is pyrrol-1-yl; or **M** is Mo; **R¹** is 2,6-diisopropylphenyl; **R²** is -C(CH₃)₂C₆H₅; **R³** is H; **R⁴** is 2,4,6-tri-t-butylphenoxy; **R⁵** is pyrrol-1-yl; or **M** is Mo; **R¹** is 2,6-diisopropylphenyl; **R²** is-C(CH₃)₂C₆H₅; **R³** is H; **R⁴** is 4-methoxy-2,3-di-t-butylphenoxy; **R⁵** is pyrrol-1-yl.

Further preferred compounds used as catalysts in the method according to the invention are the following compounds:

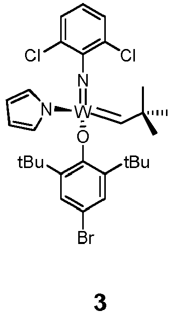

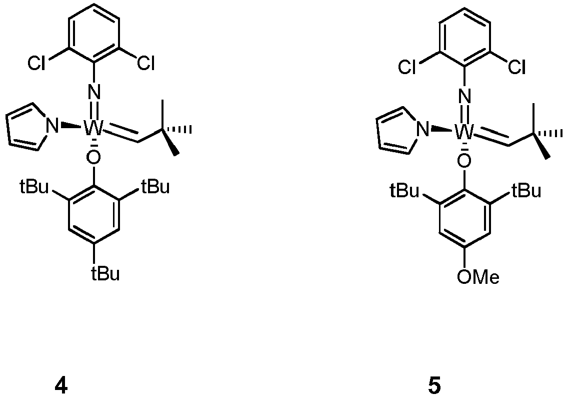

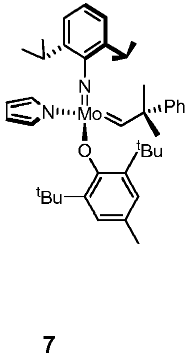

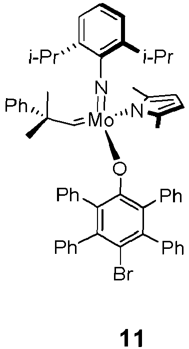

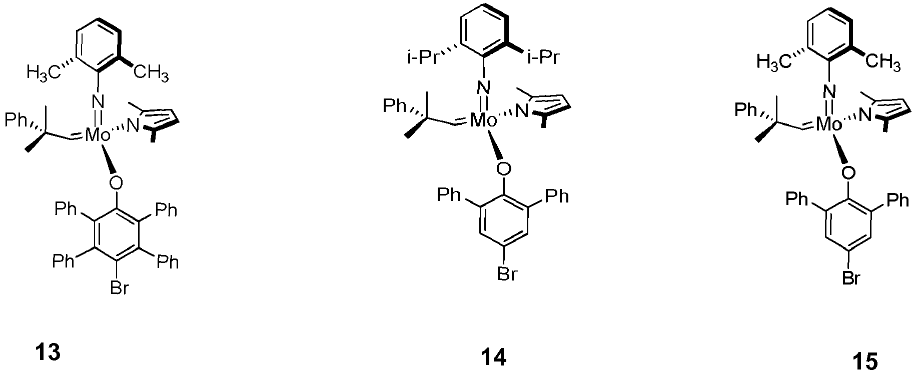

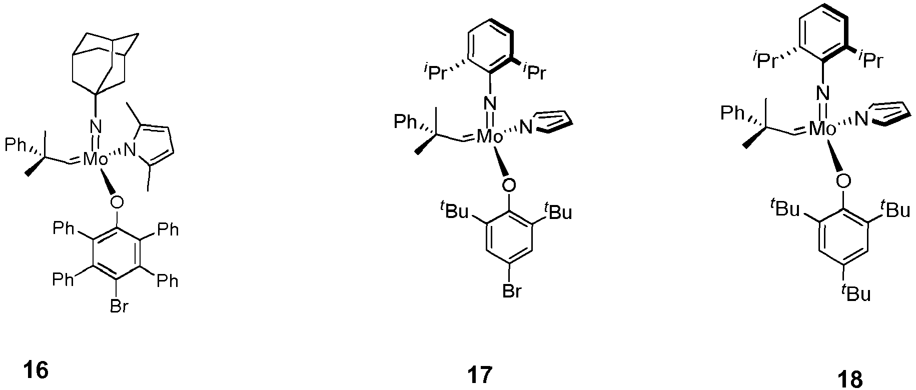

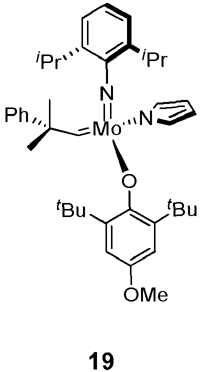

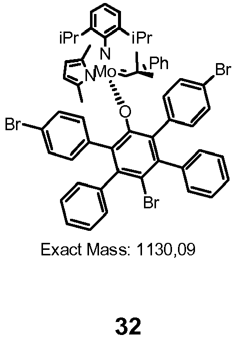

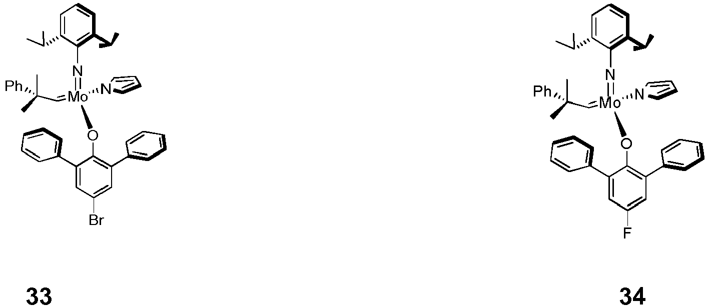

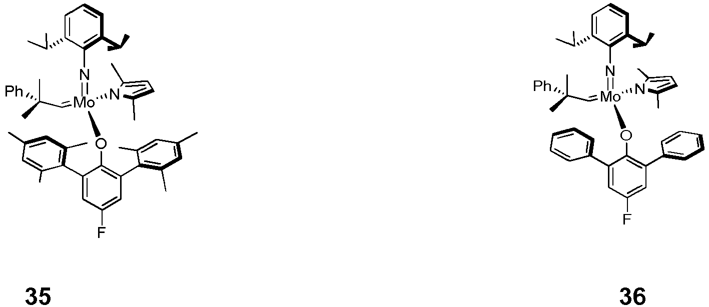

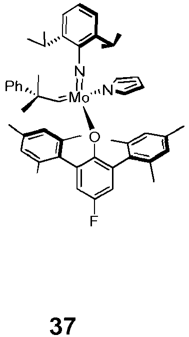

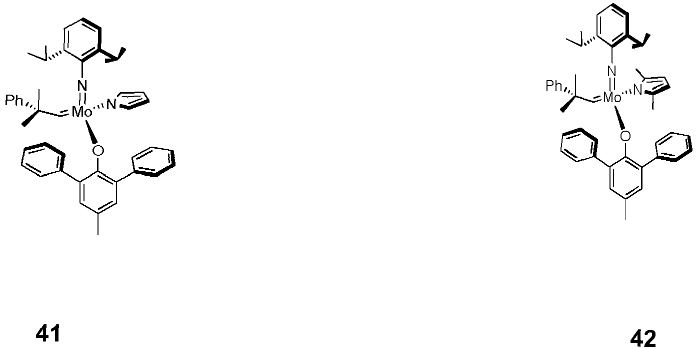

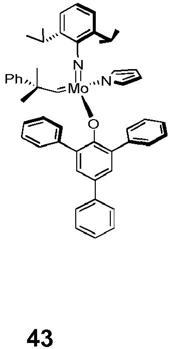

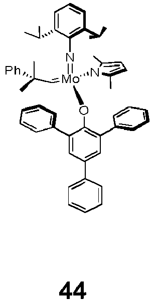

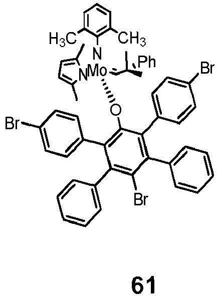

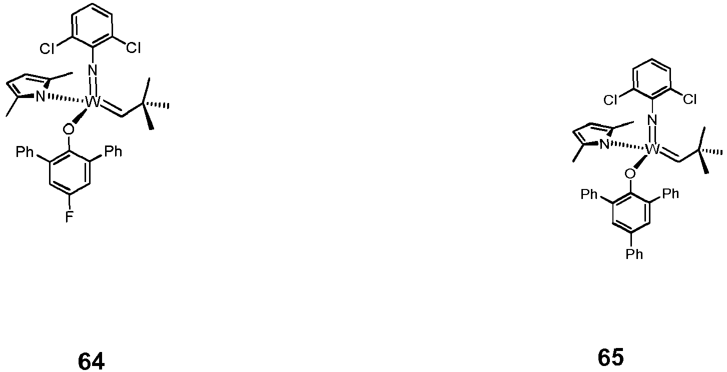

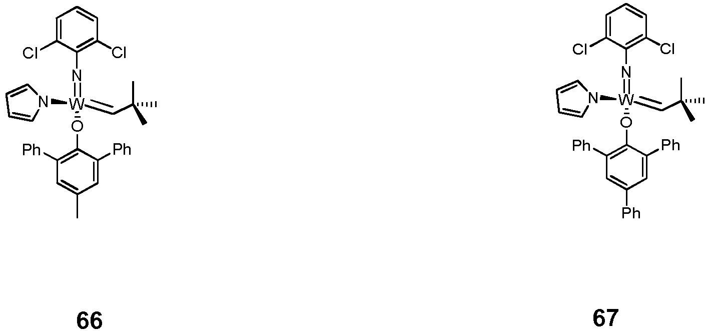

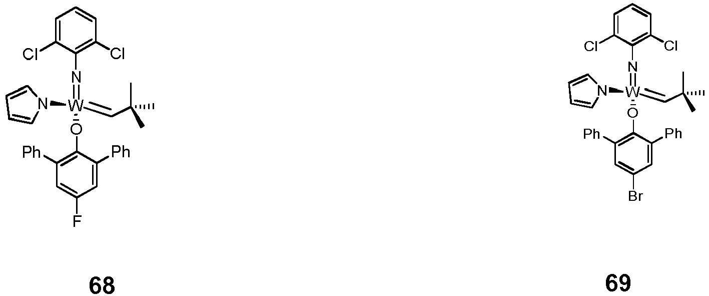

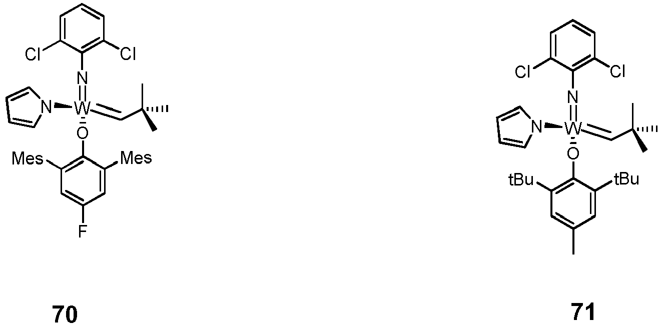

As said, in a ***first*** embodiment, the invention relates to a compound of formula 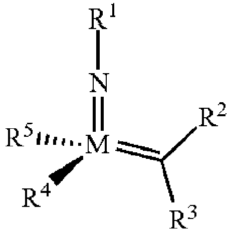 wherein **M** is Mo or W.

In one embodiment, **R¹** is selected from 2,6-dimethylphenyl, 2,6-diisopropylphenyl, 2,6-di-t-butylphenyl, 2,6-dichlorophenyl, adamant-1-yl; **R⁵** is selected from pyrrol-1-yl; 2,5-dimethyl-pyrrol-1-yl; triphenylsilyloxy; 2-phenyl-1,1,1,3,3,3-hexafluoro-prop-2-yloxy; and 9-phenyl-fluorene-9-yloxy; and **R⁴** is selected from 4-bromo-2,6-diphenylphenoxy, 4-fluoro-2,6-diphenylphenoxy, 4-methyl-2,6-diphenylphenoxy, 4-methoxy-2,6-diphenylphenoxy, 2,4,6-triphenylphenoxy, 4-fluoro-2,6-dimesitylphenoxy, 4-bromo-2,6-di-tert.-butylphenoxy, 4-methoxy-2,6-di-tert.-butylphenoxy, 4-methyl-2,6-di-tert.-butylphenoxy, 2,4,6-tri-tert.-butylphenoxy, 4-bromo-2,3,5,6-tetraphenylphenoxy, and 4-bromo-2,6-di(4-bromophenyl)-3,5-diphenylphenoxy.

The invention also relates to the use of a catalyst compound as defined above to catalyze a metathesis reaction. Accordingly, the compounds may be used as catalyst in a metathesis reaction.

In one embodiment, the metathesis reaction is selected from cross metathesis, ring opening metathesis, ring closing metathesis, ethenolysis, homo-metathesis.

Cross metathesis may e.g. be performed as a homo cross metathesis, i.e. the metathesis reaction between identical olefins (HCM) or as a hetereo cross metathesis reaction, i.e. the reaction between two different olefins.

The term *"metathesis"* refers to alkene (olefin) metathesis.

The term *"cross metathesis"* encompasses the reaction between two different olefins.

The term *"ring opening metathesis"* encompasses the ring opening of a cyclic diene.

The term *"ring closing metathesis"* encompasses the ring closing of a diene.

The term *"ethenolysis"* encompasses the reaction of an olefin having an internal olefinic bond with ethylene.

The term *"homo-metathesis"* encompasses the formation of an internal olefin from two identical terminal olefins.

The term *"conversion"* or *"conversion degree"* is defined as 100 - [final moles of first or second olefin * 100 % / initial moles of first or second olefin].

The term *"olefinic double bond"* refers to a carbon-carbon double bond or ethylenic double bond.

The term *"olefin"* refers to any species having at least one ethylenic double bond such as normal and branched chain aliphatic olefins, cycloaliphatic olefins, or aryl substituted olefins. Olefins may comprise terminal double bond(s) ("*terminal olefin*") and/or internal double bond(s) ("*internal olefin*") and can be cyclic or acyclic, linear or branched, optionally substituted. The total number of carbon atoms can be from 1 to 100, or from 1 to 40; the double bonds of a terminal olefin may be mono- or bi-substituted and the double bond of an internal olefin may be bi-, tri-, or tetrasubstituted. In some cases, an internal olefin is bisubstituted.

Non-limiting examples of molecules comprising terminal olefins are substituted and unsubstituted linear alkyl internal olefins such as C₄-C₃₀ olefins (e.g., 1-butene, 1-pentene, 1-hexene, 1-heptene, 1-octene, 1-decene, 1-dodecene, 1-tetradecene, 1-hexadecene, 1-octadecene, 1-eicosene, allylbenzene, allyltrimethylsilane, methyl-10-undecenoate, allylboronic acid pincol ester, allylbenzylether, N-allyl-4-methylbenzenesulfonamide, allylaniline, methyl-9-decenoate, allyloxy(tert-butyl)dimethyl silane, allylcyclohexane, etc.).

In one embodiment, the olefin having a terminal olefinic double bond is of formula RCH=CH₂, wherein R is selected from H, alkyl, alkenyl, aryl, heteroalkyl, heteroalkenyl, heteroaryl, or acyl, optionally substituted.

The term *"cyclic olefin"* refers to any cyclic species comprising at least one ethylenic double bond in a ring. The atoms of the ring may be optionally substituted. The ring may comprise any number of carbon atoms and/or heteroatoms. In some cases, the cyclic olefin may comprise more than one ring. A ring may comprise at least 3, at least 4, at least 5, at least 6, at least 7, at least 8, or more, atoms. Non-limiting examples of cyclic olefins include norbornene, dicyclopentadiene, bicyclo compounds, oxabicyclo compounds, and the like, all optionally substituted. *"Bicyclo compounds"* are a class of compounds consisting of two rings only, having two or more atoms in common. *"Oxabicyclo compounds"* are a class of compounds consisting of two rings only, having two or more atoms in common, wherein at least one ring comprises an oxygen atom.

## Claims

1. Compound of formula 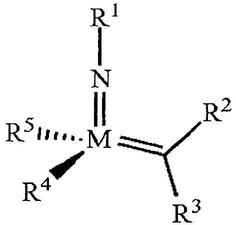 wherein
**M** is Mo or W;
**R¹** is selected from 2,6-dimethylphenyl, 2,6-diisopropylphenyl, 2,6-di-t-butylphenyl, 2,6-dichlorophenyl, adamant-1-yl;
**R²** is -C(CH₃)₂C₆H₅ or -C(CH₃)₃;
**R³** is H;
**R⁵** is selected from pyrrol-1-yl; 2,5-dimethyl-pyrrol-1-yl; triphenylsilyloxy; 2-phenyl-1,1,1,3,3,3-hexafluoro-prop-2-yloxy; 9-phenyl-fluorene-9-yloxy;
**characterized in that**
**R⁴** is selected from 4-bromo-2,6-diphenylphenoxy, 4-fluoro-2,6-diphenylphenoxy, 4-methyl-2,6-diphenylphenoxy, 4-methoxy-2,6-diphenylphenoxy, 2,4,6-triphenylphenoxy, 4-fluoro-2,6-dimesitylphenoxy, 4-bromo-2,6-di-tert.-butylphenoxy, 4-methoxy-2,6-di-tert.-butylphenoxy, 4-methyl-2,6-di-tert.-butylphenoxy, 2,4,6-tri-tert.-butylphenoxy, 4-bromo-2,3,5,6-tetraphenylphenoxy; and 4-bromo-2,6-di(4-bromophenyl)-3,5-diphenylphenoxy.

2. Compound of formula 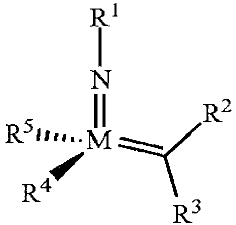 wherein
**M** is Mo or W;
**R¹** is selected from 2,6-dimethylphenyl, 2,6-diisopropylphenyl, 2,6-dichlorophenyl;
**R²** is -C(CH₃)₂C₆H₅ or -C(CH₃)₃;
**R³** is H;
**R⁵** is selected from pyrrol-1-yl; 2,5-dimethyl-pyrrol-1-yl;
**characterized in that**
**R⁴** is selected from 4-chloro-2,6-diphenylphenoxy, 4-chloro-2,3,5,6-tetraphenylphenoxy, and 4-fluoro-2,3,5,6-tetraphenylphenoxy.

3. Compound according to claim 1, wherein
**M** is Mo or W;
**R¹** is selected from 2,6-dimethylphenyl, 2,6-diisopropylphenyl, 2,6-dichlorophenyl;
**R²** is -C(CH₃)₂C₆H₅ or -C(CH₃)₃;
**R³** is H;
**R⁵** is selected from pyrrol-1-yl and 2,5-dimethyl-pyrrol-1-yl;
**R⁴** is selected from 4-methyl-2,6-di-t-butylphenoxy and 4-methoxy-2,6-di-t-butylphenoxy and 4-bromo-2,6-di-t-butylphenoxy and 2,4,6-tri-t-butylphenoxy.

4. Compound according to claim 1, wherein
**M** is W;
**R¹** is 2,6-dichlorophenyl;
**R²** is -C(CH₃)₂C₆H₅;
**R³** is H;
**R⁵** is pyrrol-1-yl;
**R⁴** is 4-bromo-2,6-di-t-butylphenoxy or 2,4,6-tri-t-butylphenoxy or 4-methoxy-2,6-di-t-butylphenoxy;
or
**M** is W;
**R¹** is 2,6-dichlorophenyl;
**R²** is-C(CH₃)₃;
**R³** is H;
**R⁵** is pyrrol-1-yl;
**R⁴** is 4-bromo-2,6-di-t-butylphenoxy or 2,4,6-tri-t-butylphenoxy or 4-methoxy-2,6-di-t-butylphenoxy.

5. Compound according to claim 1, wherein
**M** is Mo;
**R¹** is 2,6-diisopropylphenyl;
**R²** is -C(CH₃)₂C₆H₅;
**R³** is H;
**R⁵** is pyrrol-1-yl;
**R⁴** is 4-methyl-2,6-di-t-butylphenoxy or 4-bromo-2,6-di-t-butylphenoxy or 2,4,6-tri-t-butylphenoxy or 4-methoxy-2,6-di-t-butylphenoxy;
or
**M** is Mo;
**R¹** is 2,6-diisopropylphenyl;
**R²** is -C(CH₃)₃;
**R³** is H;
**R⁵** is pyrrol-1-yl;
**R⁴** is 4-methyl-2,6-di-t-butylphenoxy or 4-bromo-2,6-di-t-butylphenoxy or 2,4,6-tri-t-butylphenoxy or 4-methoxy-2,6-di-t-butylphenoxy.

6. Compound according to claim 1, wherein
**M** is Mo;
**R¹** is 2,6-diisopropylphenyl;
**R²** is -C(CH₃)₂C₆H₅;
**R³** is H;
**R⁵** is pyrrol-1-yl;
**R⁴** is 4-bromo-2,6-diphenylphenoxy or 4-fluoro-2,6-diphenylphenoxy or 4-fluoro-2,6-dimesitylphenoxy; or
**M** is Mo;
**R¹** is 2,6-diisopropylphenyl;
**R²** is -C(CH₃)₃;
**R³** is H;
**R⁵** is pyrrol-1-yl;
**R⁴** is 4-bromo-2,6-diphenylphenoxy, 4-fluoro-2,6-diphenylphenoxy, or 4-fluoro-2,6-dimesitylphenoxy.

7. Compound according to claim 1, wherein **M** is Mo;
**R¹** is 2,6-dimethylphenyl;
**R²** is -C(CH₃)₂C₆H₅ or -C(CH₃)₃;
**R³** is H;
**R⁵** is 2,5-dimethyl-pyrrol-1-yl;
**R⁴** is 4-fluoro-2,6-dimesitylphenoxy.

8. Compound according to claim 1, wherein **M** is Mo;
**R¹** is 2,6-dimethylphenyl;
**R²** is -C(CH₃)₂C₆H₅;
**R³** is H;
**R⁵** is 2,5-dimethyl-pyrrol-1-yl;
**R⁴** is selected from 4-bromo-2,6-diphenylphenoxy and 4-fluoro-2,6-dimesitylphenoxy;
or
**M** is Mo;
**R¹** is 2,6-dimethylphenyl;
**R²** is -C(CH₃)₃;
**R³** is H;
**R⁵** is 2,5-dimethyl-pyrrol-1-yl;
**R⁴** is selected from 4-bromo-2,6-diphenylphenoxy, and 4-fluoro-2,6-dimesitylphenoxy.

9. Compound according to claim 1, wherein **R¹** is 2,6-dimethylphenyl or 2,6-diisopropylphenyl;
**R²** is -C(CH₃)₂C₆H₅ or -C(CH₃)₃;
**R³** is H;
**R⁴** is 4-bromo-2,6-diphenylphenoxy or 4-bromo-2,3,5,6-tetraphenylphenoxy;
**R⁵** is pyrrol-1-yl or 2,5-dimethyl-pyrrol-1-yl.

10. Compound according to claim 1, wherein **M** is Mo;
**R¹** is 2,6-dimethylphenyl or 2,6-diisopropylphenyl;
**R²** is -C(CH₃)₂C₆H₅ or -C(CH₃)₃;
**R³** is H;
**R⁴** is 4-bromo-2,6-diphenylphenoxy;
**R⁵** is 2,5-dimethyl-pyrrol-1-yl.

11. Compound according to claim 1, wherein **M** is Mo;
**R¹** is 2,6-dimethylphenyl or 2,6-diisopropylphenyl;
**R²** is -C(CH₃)₂C₆H₅ or -C(CH₃)₃;
**R³** is H;
**R⁴** is 4-bromo-2,3,5,6-tetraphenylphenoxy;
**R⁵** is 2,5-dimethyl-pyrrol-1-yl.

12. Compound according to claim 1, wherein
**M** is Mo;
**R¹** is 2,6-diisopropylphenyl;
**R²** is -C(CH₃)₂C₆H₅ or -C(CH₃)₃;
**R³** is H;
**R⁴** is 4-methyl-2,6-di-t-butylphenoxy or 4-methoxy-2,6-di-t-butylphenoxy or 4-bromo-2,6-di-t-butylphenoxy or 2,4,6-tri-t-butylphenoxy;
**R⁵** is pyrrol-1-yl.

13. Compound according to claim 1, wherein **M** is W;
**R¹** is 2,6-dichlorophenyl;
**R²** is -C(CH₃)₃;
**R³** is H;
**R⁵** is pyrrol-1-yl;
**R⁴** is 4-methoxy-2,6-di-t-butylphenoxy or 4-bromo-2,6-di-t-butylphenoxy or 2,4,6-tri-t-butylphenoxy;
or
**M** is Mo;
**R¹** is 2,6-diisopropylphenyl;
**R²** is -C(CH₃)₂C₆H₅ or -C(CH₃)₃;
**R³** is H;
**R⁴** is 4-bromo-2,6-diphenylphenoxy or 4-bromo-2,3,5,6-tetraphenylphenoxy;
**R⁵** is pyrrol-1-yl.

14. Compound according to claim 1, wherein the compound is selected from

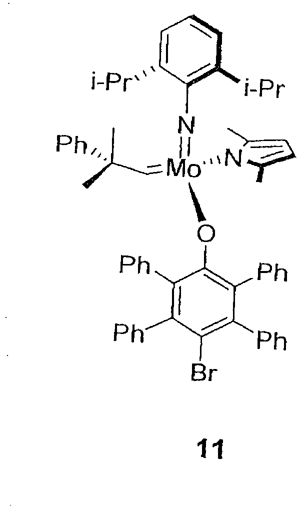

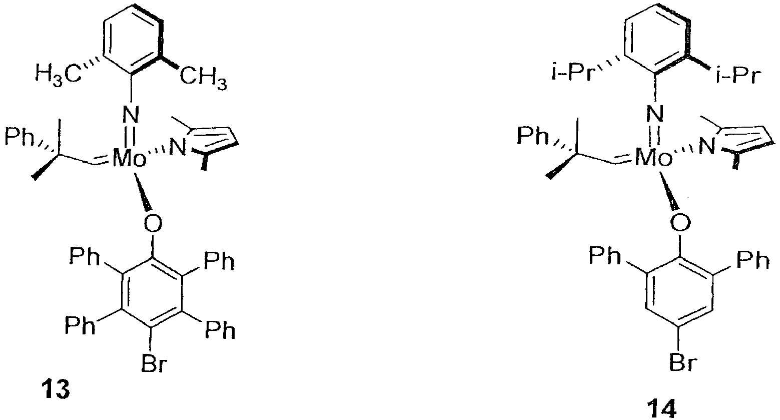

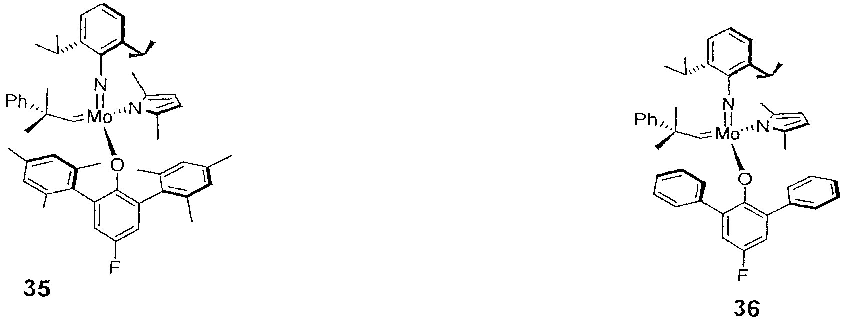

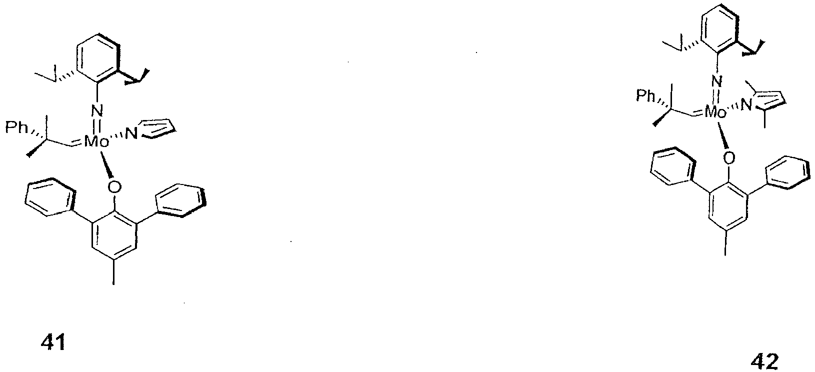

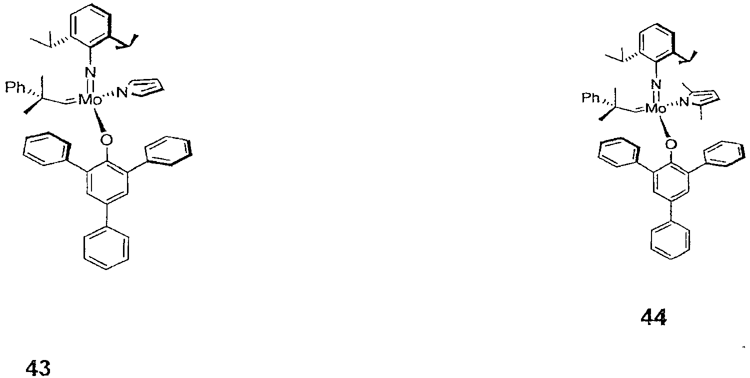

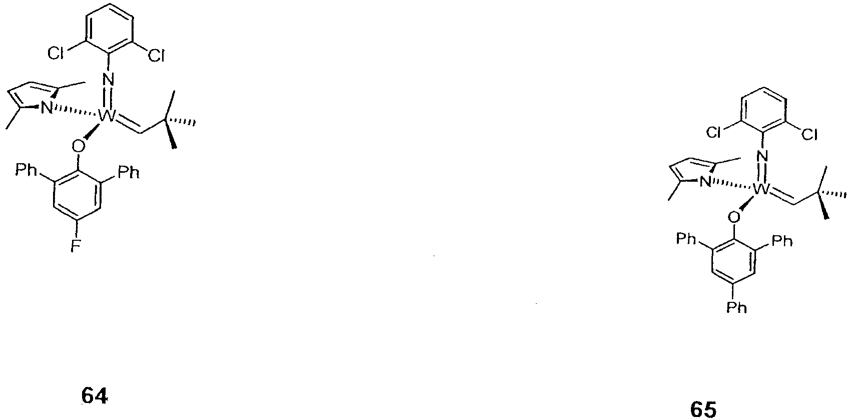

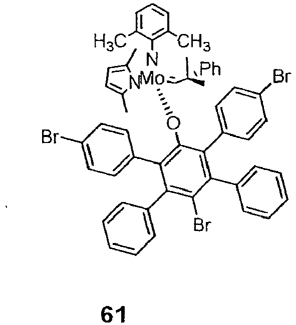

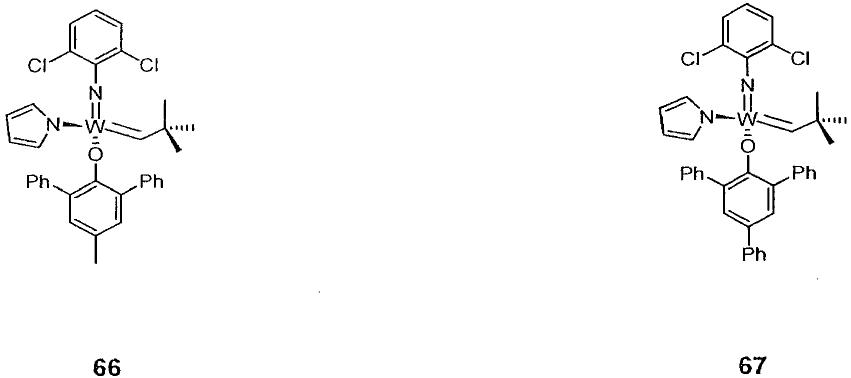

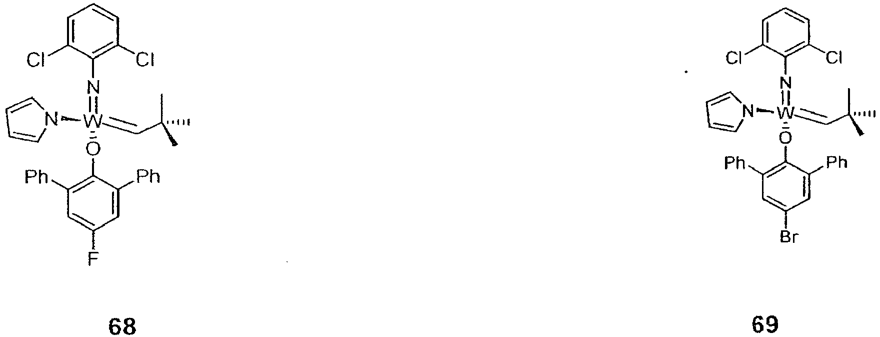

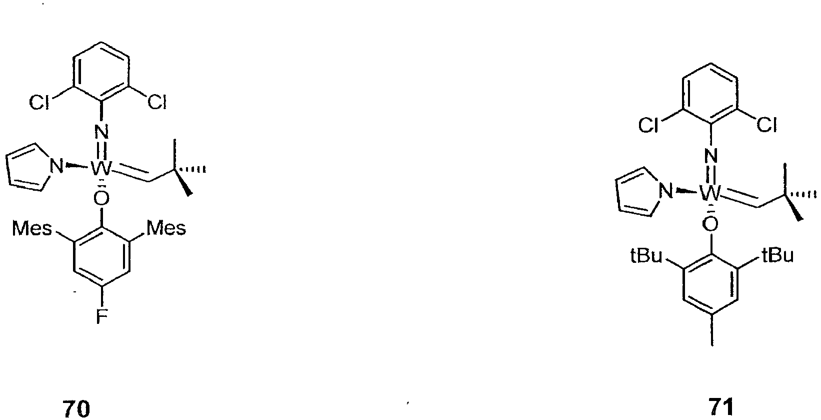

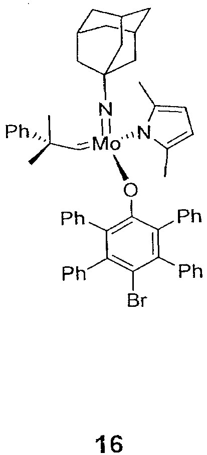

15. Use of a compound as defined in any one of the preceding claims as a catalyst in a metathesis reaction, wherein the metathesis reaction preferably is selected from cross metathesis, ring opening metathesis, ring closing metathesis, ethenolysis, or homometathesis, preferably ethenolysis.

## Patentansprüche

1. Verbindung der Formel 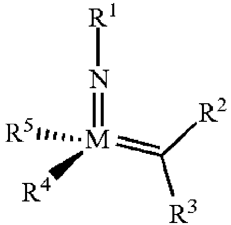 worin
**M** Mo oder W ist;
**R¹** ausgewählt ist aus 2,6-Dimethylphenyl, 2,6-Diisopropylphenyl, 2,6-Di-t-butylphenyl, 2,6-Dichlorphenyl, Adamant-1-yl;
**R²** -C(CH₃)₂C₆H₅ or -C(CH₃)₃ ist;
**R³** H ist;
**R⁵** ausgewählt ist aus Pyrrol-1-yl; 2,5-Dimethyl-pyrrol-1-yl; Triphenylsilyloxy; 2-Phenyl-1,1,1,3,3,3-hexafluor-prop-2-yloxy; 9-Phenyl-fluoren-9-yloxy;
**dadurch gekennzeichnet, dass**
**R⁴** ausgewählt ist aus 4-Brom-2,6-diphenylphenoxy, 4-Fluor-2,6-diphenylphenoxy, 4-Methyl-2,6-diphenylphenoxy, 4-Methoxy-2,6-diphenylphenoxy, 2,4,6-Triphenylphenoxy, 4-Fluor-2,6-dimesitylphenoxy, 4-Brom-2,6-di-tert.-butylphenoxy, 4-Methoxy-2,6-di-tert.-butylphenoxy, 4-Methyl-2,6-di-tert.-butylphenoxy, 2,4,6-Tri-tert.-butylphenoxy, 4-Brom-2,3,5,6-tetraphenylphenoxy und 4-Brom-2,6-di(4-bromphenyl)-3,5-diphenylphenoxy.

2. Verbindung der Formel 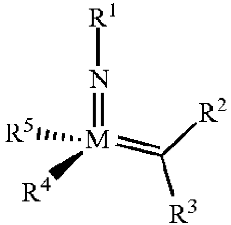 worin
**M** Mo oder W ist;
**R¹** ausgewählt ist aus 2,6-Dimethylphenyl, 2,6-Diisopropylphenyl, 2,6-Dichlorphenyl;
**R²** -C(CH₃)₂C₆H₅ oder -C(CH₃)₃ ist;
**R³** H ist;
**R⁵** ausgewählt ist aus Pyrrol-1-yl; 2,5-Dimethyl-pyrrol-1-yl;
**dadurch gekennzeichnet, dass**
**R⁴** ausgewählt ist aus 4-Chlor-2,6-diphenylphenoxy, 4-Chlor-2,3,5,6-tetraphenylphenoxy und 4-Fluor-2,3,5,6-tetraphenylphenoxy.

3. Verbindung nach Anspruch 1, wobei
**M** Mo oder W ist;
**R¹** ausgewählt ist aus 2,6-Dimethylphenyl, 2,6-Diisopropylphenyl, 2,6-Dichlorophenyl;
**R²** -C(CH₃)₂C₆H₅ oder -C(CH₃)₃ ist;
**R³** H ist;
**R⁵** ausgewählt ist aus Pyrrol-1-yl und 2,5-Dimethyl-pyrrol-1-yl;
**R⁴** ausgewählt ist aus 4-Methyl-2,6-di-t-butylphenoxy und 4-Methoxy-2,6-di-t-butylphenoxy und 4-Brom-2,6-di-t-butylphenoxy und 2,4,6-Tri-t-butylphenoxy.

4. Verbindung nach Anspruch 1, wobei
**M** W ist;
**R¹** 2,6-Dichlorphenyl ist;
**R²** -C(CH₃)₂C₆H₅ ist;
**R³** H ist;
**R⁵** Pyrrol-1-yl ist;
**R⁴** 4-Brom-2,6-di-t-butylphenoxy oder 2,4,6-Tri-t-butylphenoxy oder 4-Methoxy-2,6-di-t-butylphenoxy ist;
oder
**M** W ist;
**R¹** 2,6-Dichlorphenyl ist;
**R²** -C(CH₃)₃ ist;
**R³** H ist;
**R⁵** Pyrrol-1-yl ist;
**R⁴** 4-Brom-2,6-di-t-butylphenoxy oder 2,4,6-Tri-t-butylphenoxy oder 4-Methoxy-2,6-di-t-butylphenoxy ist.

5. Verbindung nach Anspruch 1, wobei
**M** Mo ist;
**R¹** 2,6-Diisopropylphenyl ist;
**R²** -C(CH₃)₂C₆H₅ ist;
**R³** H ist;
**R⁵** Pyrrol-1-yl ist;
**R⁴** 4-Methyl-2,6-di-t-butylphenoxy oder 4-Brom-2,6-di-t-butylphenoxy oder 2,4,6-Tri-t-butylphenoxy oder 4-Methoxy-2,6-di-t-butylphenoxy ist;
oder
**M** Mo ist;
**R¹** 2,6-Diisopropylphenyl ist;
**R²** -C(CH₃)₃ ist;
**R³** H ist;
**R⁵** Pyrrol-1-yl ist;
**R⁴** 4-Methyl-2,6-di-t-butylphenoxy oder 4-Brom-2,6-di-t-butylphenoxy oder 2,4,6-Tri-t-butylphenoxy oder 4-Methoxy-2,6-di-t-butylphenoxy ist.

6. Verbindung nach Anspruch 1, wobei
**M** Mo ist;
**R¹** 2,6-Diisopropylphenyl ist;
**R²** -C(CH₃)₂C₆H₅ ist;
**R³** H ist;
**R⁵** Pyrrol-1-yl ist;
**R⁴** 4-Brom-2,6-diphenylphenoxy oder 4-Fluor-2,6-diphenylphenoxy oder 4-Fluor-2,6-dimesitylphenoxy ist;
oder
**M** Mo ist;
**R¹** 2,6-Diisopropylphenyl ist;
**R²** -C(CH₃)₃ ist;
**R³** H ist;
**R⁵** Pyrrol-1-yl ist;
**R⁴** 4-Brom-2,6-diphenylphenoxy, 4-Fluor-2,6-diphenylphenoxy oder 4-Fluor-2,6-dimesitylphenoxy ist.

7. Verbindung nach Anspruch 1, wobei
**M** Mo ist;
**R¹** 2,6-dimethylphenyl ist;
**R²** -C(CH₃)₂C₆H₅ or -C(CH₃)₃ ist;
**R³** H ist;
**R⁵** 2,5-Dimethyl-pyrrol-1-yl ist;
**R⁴** 4-Fluor-2,6-dimesitylphenoxy ist.

8. Verbindung nach Anspruch 1, wobei
**M** Mo ist;
**R¹** 2,6-Dimethylphenyl ist;
**R²** -C(CH₃)₂C₆H₅ ist;
**R³** H ist;
**R⁵** 2,5-Dimethyl-pyrrol-1-yl ist;
**R⁴** ausgewählt ist aus 4-Brom-2,6-diphenylphenoxy und 4-Fluor-2,6-dimesitylphenoxy;
oder
**M** Mo ist;
**R¹** 2,6-Dimethylphenyl ist;
**R²** -C(CH₃)₃ ist;
**R³** H ist;
**R⁵** 2,5-Dimethyl-pyrrol-1-yl ist;
**R⁴** ausgewählt ist aus 4-Brom-2,6-diphenylphenoxy und 4-Fluor-2,6-dimesitylphenoxy.

9. Verbindung nach Anspruch 1, wobei
**R¹** 2,6-Dimethylphenyl or 2,6-diisopropylphenyl ist;
**R²** -C(CH₃)₂C₆H₅ or -C(CH₃)₃ ist;
**R³** H ist;
**R⁴** 4-Brom-2,6-diphenylphenoxy oder 4-Brom-2,3,5,6-tetraphenylphenoxy ist;
**R⁵** Pyrrol-1-yl oder 2,5-Dimethyl-pyrrol-1-yl ist.

10. Verbindung nach Anspruch 1, wobei
**M** Mo ist;
**R¹** 2,6-Dimethylphenyl oder 2,6-Diisopropylphenyl ist;
**R²** -C(CH₃)₂C₆H₅ or -C(CH₃)₃ ist;
**R³** H ist;
**R⁴** 4-Brom-2,6-diphenylphenoxy ist;
**R⁵** 2,5-Dimethyl-pyrrol-1-yl ist.

11. Verbindung nach Anspruch 1, wobei
**M** Mo ist;
**R¹** 2,6-Dimethylphenyl oder 2,6-Diisopropylphenyl ist;
**R²** -C(CH₃)₂C₆H₅ oder-C(CH₃)₃ ist;
**R³** H ist;
**R⁴** 4-Brom-2,3,5,6-tetraphenylphenoxy ist;
**R⁵** 2,5-Dimethyl-pyrrol-1-yl ist.

12. Verbindung nach Anspruch 1, wobei
**M** Mo ist;
**R¹** 2,6-Diisopropylphenyl ist;
**R²** -C(CH₃)₂C₆H₅ or -C(CH₃)₃ ist;
**R³** H ist;
**R⁴** 4-Methyl-2,6-di-t-butylphenoxy oder 4-Methoxy-2,6-di-t-butylphenoxy oder 4-Brom-2,6-di-t-butylphenoxy oder 2,4,6-Tri-t-butylphenoxy ist;
**R⁵** Pyrrol-1-yl ist.

13. Verbindung nach Anspruch 1, wobei
**M** W ist;
**R¹** 2,6-Dichlorphenyl ist;
**R²** -C(CH₃)₃ ist;
**R³** H ist;
**R⁵** Pyrrol-1-yl ist;
**R⁴** 4-Methoxy-2,6-di-t-butylphenoxy oder 4-Brom-2,6-di-t-butylphenoxy oder 2,4,6-Tri-t-butylphenoxy ist;
oder
**M** Mo ist;
**R¹** 2,6-Diisopropylphenyl ist;
**R²** -C(CH₃)₂C₆H₅ or -C(CH₃)₃ ist;
**R³** H ist;
**R⁴** 4-Brom-2,6-diphenylphenoxy oder 4-Brom-2,3,5,6-tetraphenylphenoxy ist;
**R⁵** Pyrrol-1-yl ist.

14. Verbindung nach Anspruch 1, wobei die Verbindung ausgewählt ist aus

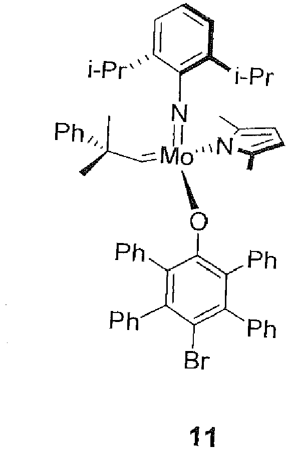

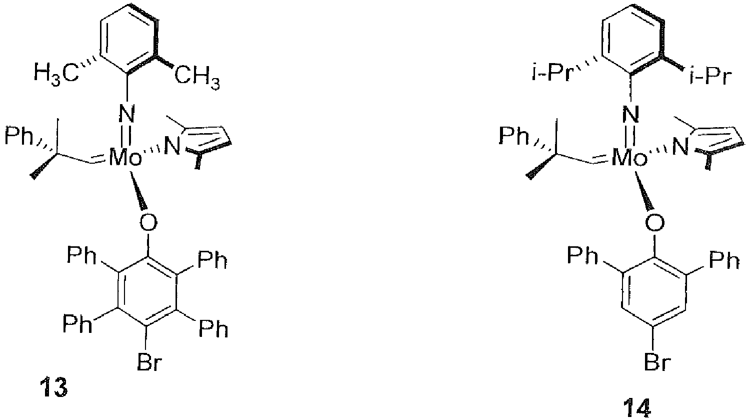

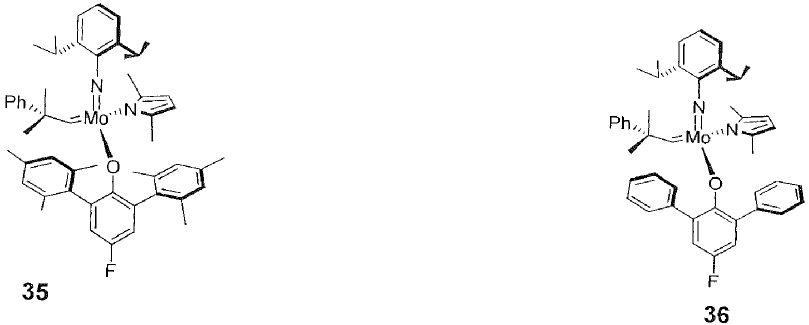

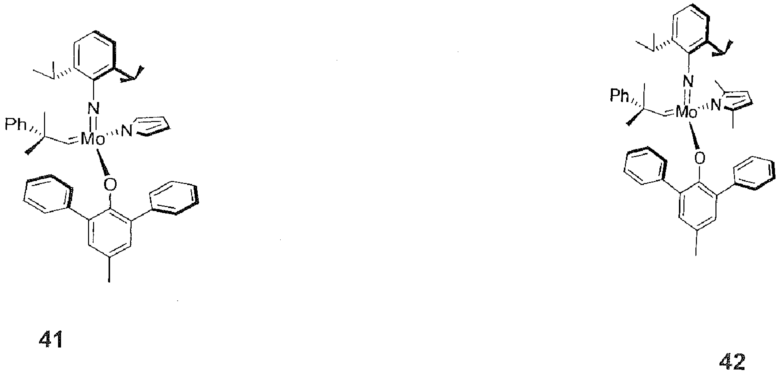

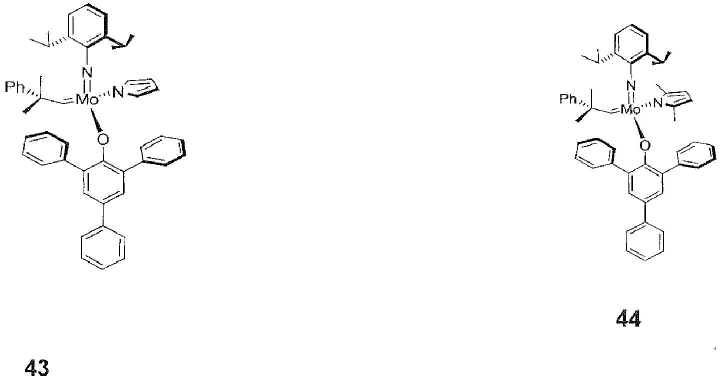

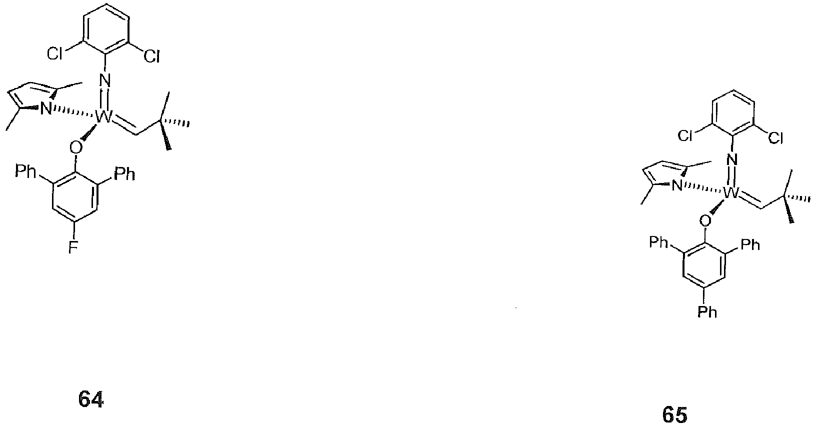

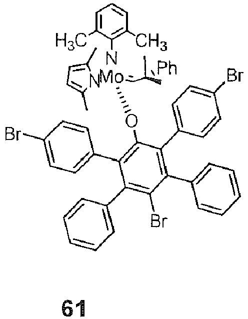

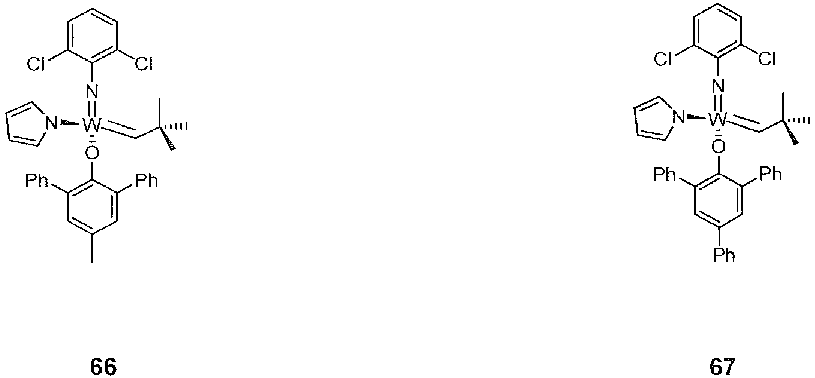

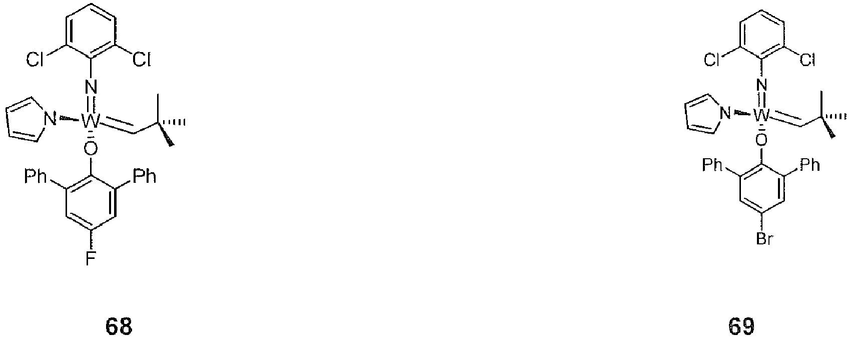

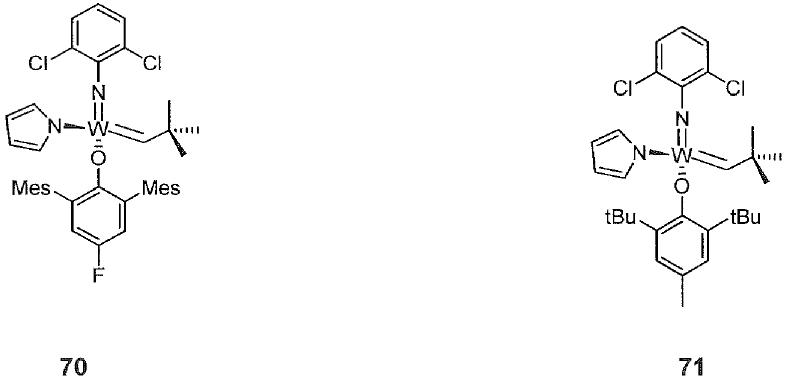

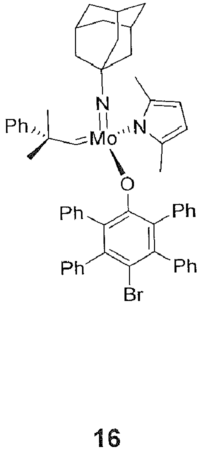

15. Verwendung einer Verbindung wie in einem der vorstehenden Ansprüche definiert als Katalysator in einer Metathesereaktion, wobei die Metathesereaktion vorzugsweise ausgewählt ist aus Kreuzmetathese, Ring-öffnender Metathese, Ringschließender Metathese, Ethenolyse, oder Homometathese, vorzugsweise Ethenolyse.

## Revendications

1. Composé de formule 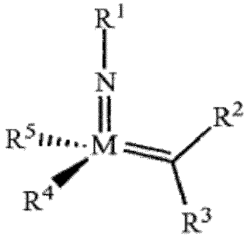 dans lequel
**M** est Mo ou W ;
**R¹** est choisi parmi 2,6-diméthylphényl, 2,6-diisopropylphényl, 2,6-di-t-butylphényl, 2,6-dichlorophényl, adamant-1-yl ;
**R²** est -C(CH₃)₂C₆H₅ ou -C(CH₃)₃ ;
**R³** est H ;
**R⁵** est choisi parmi pyrrol-1-yl; 2,5-diméthyl-pyrrol-1-yl; triphénylsilyloxy; 2-phényl-1,1,1,3,3,3-hexafluoro-prop-2-yloxy ; 9-phényl-fluorene-9-yloxy ; **caractérisé en ce que**
**R⁴** est choisi parmi 4-bromo-2,6-diphénylphénoxy, 4-fluoro-2,6-diphénylphénoxy,
4-méthyl-2,6-diphénylphénoxy, 4-méthoxy-2,6-diphénylphénoxy, 2,4,6-triphénylphénoxy, 4-fluoro-2,6-dimesitylphénoxy, 4-bromo-2,6-di-tert.-butylphénoxy, 4-méthoxy-2,6-di-tert.-butylphenoxy, 4-méthyl-2,6-di-tert.-butylphénoxy, 2,4,6-tri-tert.-butylphénoxy, 4-bromo-2,3,5,6-tetraphénylphénoxy ; et 4-bromo-2,6-di(4-bromophényl)-3,5-diphénylphénoxy.

2. Composé de formule 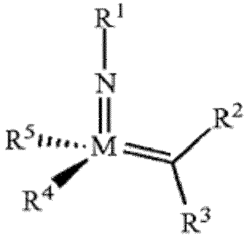 dans lequel
**M** est Mo ou W ;
**R¹** est choisi parmi 2,6-diméthylphényl, 2,6-diisopropylphényl, 2,6-dichlorophényl;
**R²** est -C(CH₃)₂C₆H₅ ou -C(CH₃)₃ ;
**R³** est H ;
**R⁵** est choisi parmi pyrrol-1-yl ; 2,5-diméthyl-pyrrol-1-yl ;
**caractérisé en ce que**
**R⁴** est choisi parmi 4-chloro-2,6-diphénylphénoxy, 4-chloro-2,3,5,6-tetraphénylphénoxy, et 4-fluoro-2,3,5,6-tetraphénylphénoxy.

3. Composé selon la revendication 1, dans lequel
**M** est Mo ou W ;
**R¹** est choisi parmi 2,6-diméthylphényl, 2,6-diisopropylphényl, 2,6-dichlorophényl ;
**R²** est -C(CH₃)₂C₆H₅ ou -C(CH₃)₃ ;
**R³** est H ;
**R⁵** est choisi parmi pyrrol-1-yl ; 2,5-diméthyl-pyrrol-1-yl ;
**R⁴** est choisi parmi 4-méthyl-2,6-di-t-butylphénoxy et 4-méthoxy-2,6-di-t-butylphénoxy et 4-bromo-2,6-di-t-butylphénoxy et 2,4,6-tri-t-butylphénoxy.

4. Composé selon la revendication 1, dans lequel
**M** est W ;
**R¹** est 2,6-dichlorophényl ;
**R²** est -C(CH₃)₂C₆H₅ ;
**R³** est H ;
**R⁵** est pyrrol-1-yl ;
**R⁴** est 4-bromo-2,6-di-t-butylphénoxy ou 2,4,6-tri-t-butylphénoxy ou 4-méthoxy-2,6-di-t-butylphénoxy ;
ou
**M** est W ;
**R¹** est 2,6-dichlorophényl ;
**R²** est -C(CH₃)₃ ;
**R³** est H ;
**R⁵** est pyrrol-1-yl ;
**R⁴** est 4-bromo-2,6-di-t-butylphénoxy ou 2,4,6-tri-t-butylphénoxy ou 4-méthoxy-2,6-di-t-butylphénoxy.

5. Composé selon la revendication 1, dans lequel
**M** est Mo ;
**R¹** est 2,6-diisopropylphényl ;
**R²** est -C(CH₃)₂C₆H₅ ;
**R³** est H ;
**R⁵** est pyrrol-1-yl ;
**R⁴** est 4-méthyl-2,6-di-t-butylphénoxy ou 4-bromo-2,6-di-t-butylphénoxy ou 2,4,6-tri-t-butylphénoxy ou 4-méthoxy-2,6-di-t-butylphénoxy ;
ou
**M** est Mo ;
**R¹** est 2,6-diisopropylphényl ;
**R²** est -C(CH₃)₃ ;
**R³** est H ;
**R⁵** est pyrrol-1-yl ;
**R⁴** est 4-méthyl-2,6-di-t-butylphénoxy ou 4-bromo-2,6-di-t-butylphénoxy ou 2,4,6-tri-t-butylphénoxy ou 4-méthoxy-2,6-di-t-butylphénoxy.

6. Composé selon la revendication 1, dans lequel
**M** est Mo ;
**R¹** est 2,6-diisopropylphényl ;
**R²** est -C(CH₃)₂C₆H₅ ;
**R³** est H ;
**R⁵** est pyrrol-1-yl ;
**R⁴** est 4-bromo-2,6-diphénylphénoxy ou 4-fluoro-2,6-diphénylphénoxy ou 4-fluoro-2,6-dimesitylphénoxy ;
ou
**M** est Mo ;
**R¹** est 2,6-diisopropylphényl ;
**R²** est -C(CH₃)₃ ;
**R³** est H ;
**R⁵** est pyrrol-1-yl ;
**R⁴** est 4-bromo-2,6-diphénylphénoxy, 4-fluoro-2,6-diphénylphénoxy, ou 4-fluoro-2,6-dimesitylphénoxy.

7. Composé selon la revendication 1, dans lequel
**M** est Mo ;
**R¹** est 2,6-diméthylphényl ;
**R²** est -C(CH₃)₂C₆H₅ ou -C(CH₃)₃ ;
**R³** est H ;
**R⁵** est 2,5-diméthyl-pyrrol-1-yl ;
**R⁴** est 4-fluoro-2,6-dimesitylphénoxy.

8. Composé selon la revendication 1, dans lequel
**M** est Mo ;
**R¹** est 2,6-diméthylphényl ;
**R²** est -C(CH₃)₂C₆H₅ ;
**R³** est H ;
**R⁵** est 2,5-diméthyl-pyrrol-1-yl ;
**R⁴** est choisi parmi 4-bromo-2,6-diphénylphénoxy et 4-fluoro-2,6-dimesitylphénoxy ;
ou
**M** est Mo ;
**R¹** est 2,6-diméthylphényl ;
**R²** est -C(CH₃)₃ ;
**R³** est H ;
**R⁵** est 2,5-diméthyl-pyrrol-1-yl ;
**R⁴** est choisi parmi 4-bromo-2,6-diphénylphénoxy, et 4-fluoro-2,6-dimesitylphénoxy.

9. Composé selon la revendication 1, dans lequel
**R¹** est 2,6-diméthylphényl ou 2,6-diisopropylphényl ;
**R²** est -C(CH₃)₂C₆H₅ ou -C(CH₃)₃ ;
**R³** est H ;
**R⁴** est 4-bromo-2,6-diphénylphénoxy ou 4-bromo-2,3,5,6-tetraphénylphénoxy ; **R⁵** est pyrrol-1-yl ou 2,5-diméthyl-pyrrol-1-yl.

10. Composé selon la revendication 1, dans lequel
**M** est Mo ;
**R¹** est 2,6-diméthylphényl ou 2,6-diisopropylphényl ;
**R²** est -C(CH₃)₂C₆H₅ ou -C(CH₃)₃ ;
**R³** est H ;
**R⁴** est 4-bromo-2,6-diphénylphénoxy ;
**R⁵** est 2,5-diméthyl-pyrrol-1-yl.

11. Composé selon la revendication 1, dans lequel
**M** est Mo ;
**R¹** est 2,6-diméthylphényl ou 2,6-diisopropylphényl ;
**R²** est -C(CH₃)₂C₆H₅ ou -C(CH₃)₃ ;
**R³** est H ;
**R⁴** est 4-bromo-2,3,5,6-tetraphénylphénoxy ;
**R⁵** est 2,5-diméthyl-pyrrol-1-yl.

12. Composé selon la revendication 1, dans lequel
**M** est Mo ;
**R¹** est 2,6-diisopropylphényl ;
**R²** est -C(CH₃)₂C₆H₅ ou -C(CH₃)₃ ;
**R³** est H ;
**R⁴** est 4-méthyl-2,6-di-t-butylphénoxy ou 4-méthoxy-2,6-di-t-butylphénoxy ou 4-bromo-2,6-di-t-butylphénoxy ou 2,4,6-tri-t-butylphénoxy ;
**R⁵** est pyrrol-1-yl.

13. Composé selon la revendication 1, dans lequel
**M** est W ;
**R¹** est 2,6-dichlorophényl ;
**R²** est - C(CH₃)₃ ;
**R³** est H ;
**R⁵** est pyrrol-1-yl ;
**R⁴** est 4-méthoxy-2,6-di-t-butylphénoxy ou 4-bromo-2,6-di-t-butylphénoxy ou 2,4,6-tri-t-butylphénoxy.
ou
**M** est Mo ;
**R¹** est 2,6-diisopropylphényl ;
**R²** est C(CH₃)₂C₆H₅ ou -C(CH₃)₃ ;
**R³** est H ;
**R⁴** est 4-bromo-2,6-diphénylphénoxy ou 4-bromo-2,3,5,6-tetraphénylphénoxy ;
**R⁵** est pyrrol-1-yl.

14. Composé selon la revendication 1, dans lequel le composé est choisi parmi

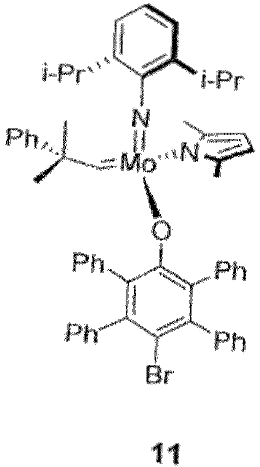

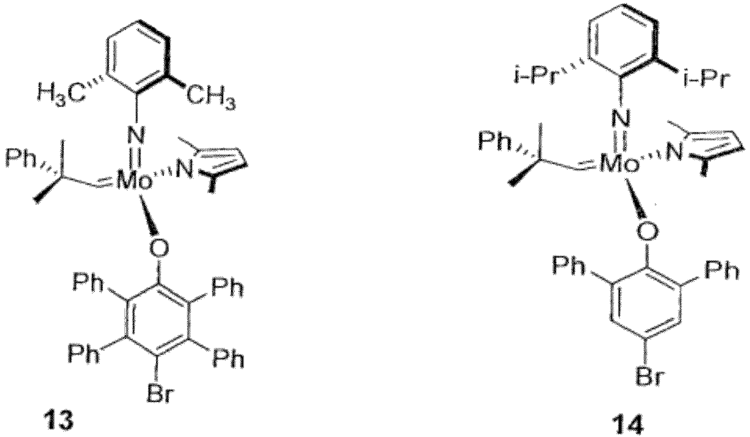

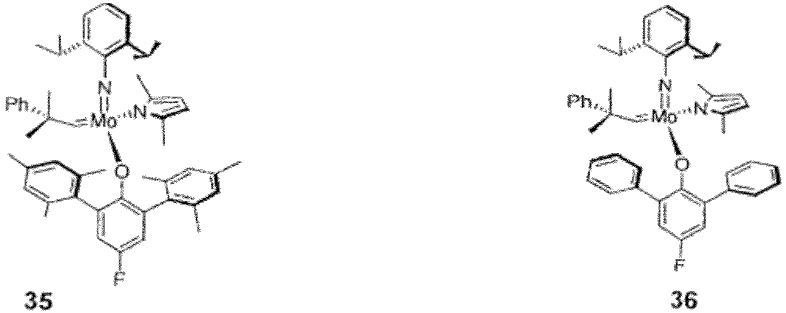

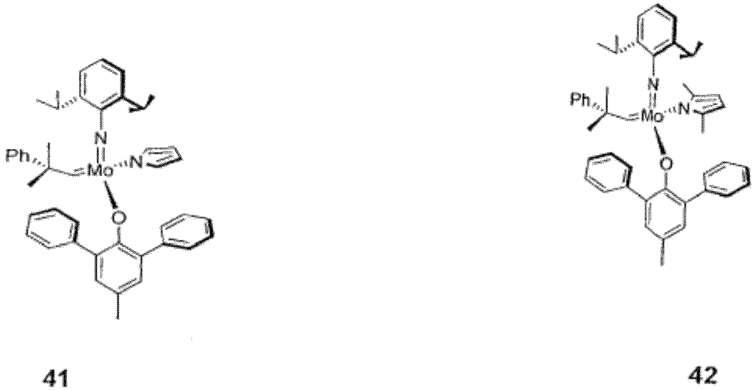

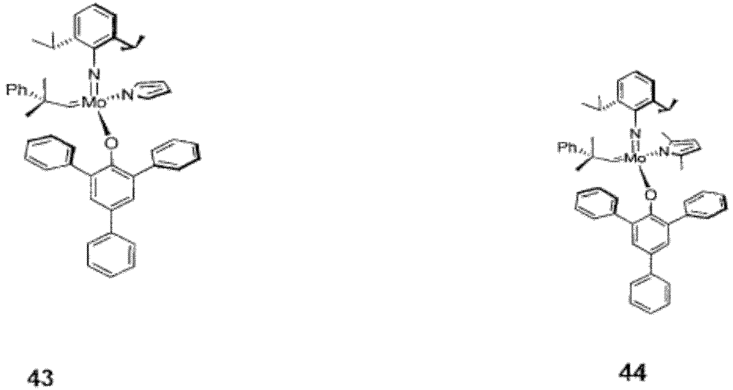

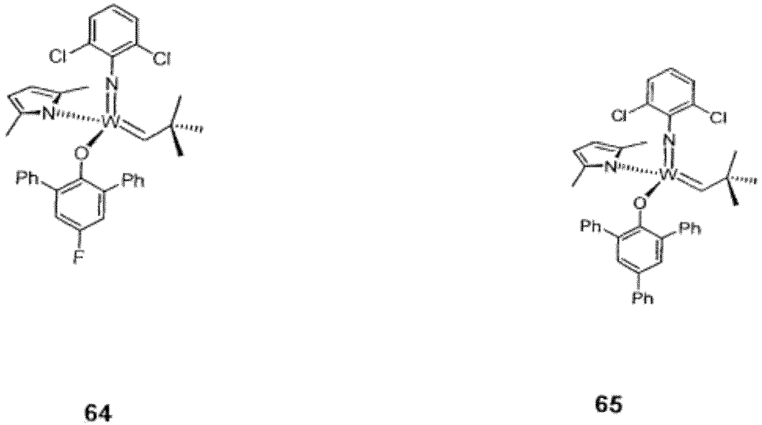

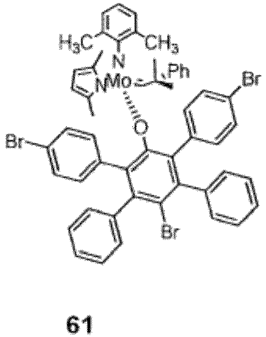

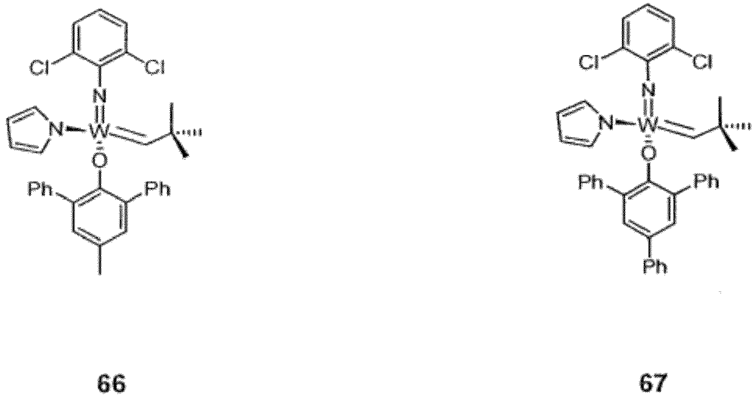

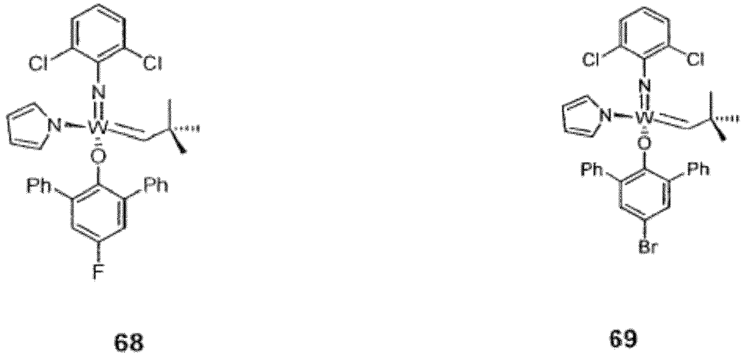

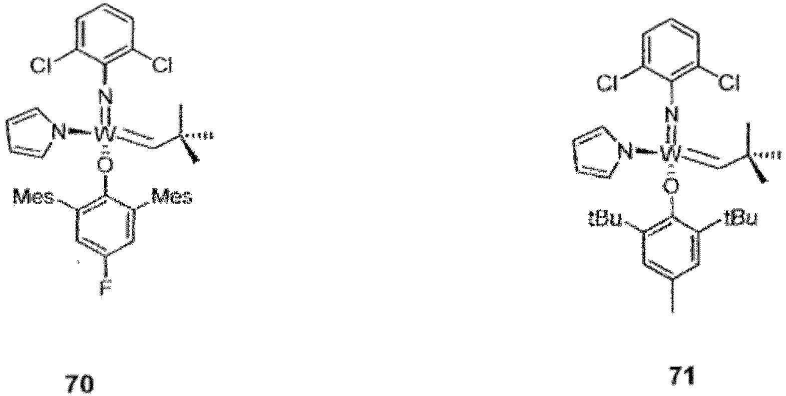

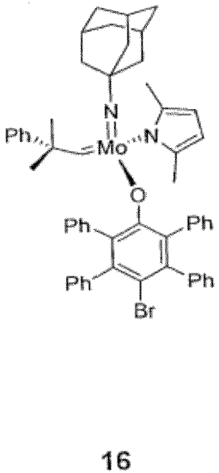

15. Utilisation d'un composé tel que défini selon l'une quelconque des revendications précédentes comme un catalyseur dans une réaction de métathèse, dans laquelle la réaction de métathèse est préférentiellement choisie parmi métathèse croisée, métathèse à ouverture de cycle, métathèse de cyclisation, éthénolyse, ou homométathèse, préférentiellement éthénolyse.
